# DEMANDE DE BREVET EUROPEEN

(11) **EP 1 488 765 A1**
(43) Date de publication de la demande: **22.12.2004**
(21) Numéro de dépôt: 04356105.9
(22) Date de dépôt: 18.06.2004
(51) Int. Cl.: A61F 5/055

(54) **Collier cervical à soutien variable**

(30) Priorité: 18.06.2003 FR 0307353
(71) Demandeur: Gibaud, F-42000 Saint Etienne (FR)
(72) Inventeur: Vernay, Philippe, 42000 Saint Etienne (FR)
(74) Mandataire: Delorme, Nicolas

(57) **Abrégé**

Le collier (1) cervical selon l'invention comprend une âme (2) en matériau élastique et des moyens d'accrochage aptes à maintenir le collier autour du cou d'un patient.

Selon l'invention, l'âme présente au moins un logement dans lequel sont intégrés des moyens de sélection d'un module d'élasticité du collier parmi au moins deux modules distincts.

## Description

La présente invention concerne un collier cervical à maintien régiabie.

Pour le traitement de pathologies cervicales telles que torticolis ou crise de cervicarthrose, il est bien connu d'utiliser un collier cervical qui est enroulé autour du cou d'un patient.

De manière classique, un collier cervical comprend une âme en mousse insérée dans une gaine textile. Le maintien du collier autour du cou du patient se fait généralement par une bande de tissu auto agrippant.

Les colliers de ce type qui agissent, d'une part, par une immobilisation partielle de la région cervicale, et d'autre part, par un effet thermique, ne sont toutefois pas totalement satisfaisants.

En effet, selon l'évolution de la pathologie ou selon la posture du patient (debout ou couché), il est souhaitable de faire varier le maintien que le collier exerce dans la région cervicale.

Or, ce maintien est fixé par la densité de la mousse constituant l'âme du collier.

On connaît par le document EP-A-923 917 un collier cervical en mousse élastique qui comprend des éléments de stabilisation qui agissent à certains points du collier, mais ne permettent pas un réglage par le patient du maintien conféré par le collier.

Le document US 5 904 662 montre un collier cervical qui présente des ouvertures dans lesquelles il est possible d'installer des bouchons ou des vessies gonflables. Un inconvénient de ce collier réside dans le fait qu'il nécessite des accessoires (bouchons amovibles, moyens de gonflage des vessies) pour permettre au patient de régler le maintien procuré par le collier.

Un but de l'invention est donc de proposer un collier cervical dont le maintien puisse être réglé, de manière autonome, par le patient en fonction de paramètres tels que son état ou sa position.

De manière connue en soi, le collier cervical comprend une âme en matériau élastique et des moyens d'accrochage aptes à maintenir le collier autour du cou d'un patient.

Selon l'invention, l'âme présente au moins un logement dans lequel sont intégrés des moyens de sélection d'un module d'élasticité du collier parmi au moins deux modules distincts.

Grâce à cet insert, le patient peut, en fonction de l'état de sa pathologie ou en fonction de sa position, faire varier le module d'élasticité du collier cervical qu'il porte.

En d'autres termes, il peut, en agissant, sur les moyens de sélection opérer une sélection entre plusieurs configurations de maintien.

Ces configurations de maintien sont, au moins, une configuration dans laquelle les moyens de sélection permettent de conférer au collier un maintien souple, c'est-à-dire dans laquelle le collier peut se déformer de manière importante et une configuration dans laquelle les moyens de sélection permettent de conférer au collier un maintien ferme, c'est-à-dire dans laquelle le collier se déforme peu et offre donc un maintien important au niveau de la région cervicale.

Un point qu'il est important de souligner est que le collier, selon l'invention, est totalement autonome et ne nécessite pas d'accessoires externes pour conférer au patient plusieurs configurations de maintien.

Les moyens de sélection sont entièrement intégrés au collier ; aucun accessoire externe n'est nécessaire pour réaliser un réglage du maintien contrairement aux dispositifs de l'art antérieur.

Selon plusieurs caractéristiques que peut prendre le collier :
- le logement est une ouverture circulaire pratiquée dans l'épaisseur du collier dans lequel s'engage l'insert,
- l'insert est susceptible d'entrer en rotation dans l'ouverture pour passer d'une orientation de l'insert conférant au collier un premier module d'élasticité à au moins une deuxième orientation de l'insert conférant au collier un deuxième module d'élasticité,
- l'insert est muni d'un bouton dépassant d'une surface latérale de l'âme.

Dans une première forme de réalisation de l'insert, l'insert est constitué d'une paroi diamétrale portant, à chaque extrémité, deux parois périphériques en arc de cercle. De plus, les parois périphériques en arc de cercle sont munies à chacune de leurs extrémités d'un retour dirigé vers l'intérieur de l'insert.

Dans une deuxième forme de réalisation de l'insert, l'insert est constitué d'une paroi périphérique cylindrique et de deux parois perpendiculaires.

Dans une troisième forme de réalisation de l'insert, l'insert est constitué de deux parois diagonales et deux parois périphériques en arc de cercle opposées l'une à l'autre par rapport à la ligne d'intersection des parois diagonales.

Dans une quatrième forme de réalisation de l'insert, l'insert est constitué d'une paroi diamétrale portant à chacune de ses extrémités deux parois périphériques en arc de cercle s'étendant chacun sur au moins 180°. Selon une possibilité, des éléments de mousse sont engagés dans les espaces délimités pour les parois de chaque insert.

Dans une cinquième forme de réalisation de l'insert, l'insert est constitué d'une paroi périphérique cylindrique et d'un disque relié à la paroi cylindrique par deux ponts de matière diamétralement opposés, le disque présentant au moins deux coefficients d'élasticité.

Selon plusieurs caractéristiques de cette forme de réalisation de l'insert :
- le disque présente au moins une ondulation passant par le centre du disque et perpendiculaire au plan passant par les deux ponts de matière,
- le disque présente trois ondulations parallèles.

Dans une sixième forme de réalisation, l'insert est constitué d'une paroi périphérique cylindrique périphérique de laquelle s'étendent radialement trois nervures rigides disposées à 120° et trois lames élastiques auxquelles est relié un noyau mobile le long de l'axe de l'insert entre une position dans laquelle le noyau est en appui contre les nervures rigides et une position dans laquelle le noyau est dégagé de ces dernières et est uniquement retenu par les lames souples.

Selon une caractéristique avantageuse, la paroi périphérique de l'insert est munie d'oreilles permettant de retenir l'insert par rapport à l'âme.

De plus, une gaine de matière textile recouvre l'âme.

Pour sa bonne compréhension, l'invention est décrite en référence au dessin ci annexé représentant, à titre d'exemple non limitatif, un collier cervical à maintien réglable et plusieurs formes de réalisation et de moyens permettant le réglage de son maintien.
Figure 1 représente en perspective un collier cervical avec arrachement partiel de la gaine textile,
Figures 2 à 5représentent plusieurs formes de réalisation d'un insert destiné à être logé dans le collier,
Figures 6 et 7 représentent, respectivement, en perspective et en vue de face, une autre forme de réalisation de l'insert,
Figures 8 et 9 représentent, en coupe, l'insert de figures 3 et 4 selon deux orientations dans chacune desquelles l'insert présente une élasticité spécifique,
Figures 10 à 12 représentent respectivement, en perspective et en coupe, une forme de réalisation de l'insert.

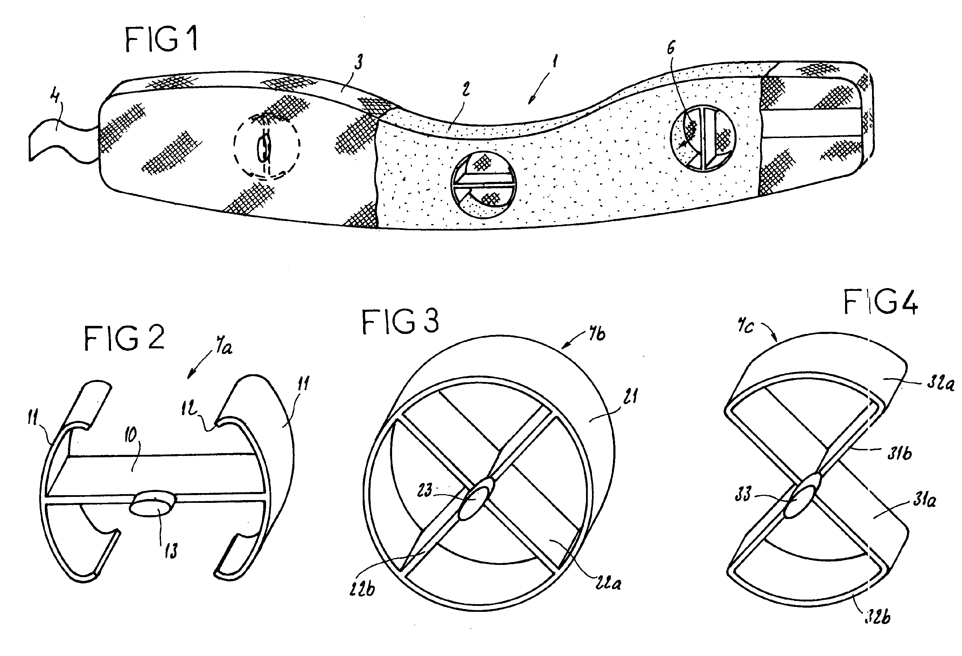

En se référant tout d'abord à la figure 1, on peut voir que le collier 1 cervical comprend, de manière classique, une âme 2 en mousse insérée dans une gaine 3 en matière textile. La fixation du collier sur le cou d'un patient se fait par un ruban 4 de textile auto agrippant. On comprend que le maintien d'un tel collier est conféré par la nature du matériau constituant l'âme 2. Il s'agit d'une mousse qui présente une élasticité donnée. Cette élasticité est donc invariable.

La figure 1 montre que, dans l'âme 2 du collier, des découpes sont pratiquées dans l'épaisseur de l'âme (par épaisseur, on entend la plus petite des dimensions de l'âme). Ces découpes sont au nombre de trois correspondants sensiblement lorsque le collier est placé sur le cou d'un patient à la région du menton et aux régions latérales participant au maintien occipital.

Ces découpes sont des ouvertures 6 circulaires d'un diamètre d'environ cinquante millimètres. Dans chaque ouverture 6 est engagé un insert qui permet de conférer au collier des modules d'élasticité différents que le patient peut sélectionner en fonction du maintien qu'il souhaite obtenir du collier cervical.

La figure 1 représente une première forme de réalisation de cet insert.

Comme on peut le voir sur cette figure, l'insert 7a est constitué d'une paroi diamétrale 10 et, à chaque extrémité de cette paroi diamétrale 10, deux parois périphériques en arc de cercle 11. On note également qu'un bouton de manoeuvre 13 est placé sur la paroi diamétrale au niveau de l'axe de l'insert. On note également que les extrémités de chacune des parois périphériques en arc de cercle 11 sont pourvues d'un retour 12 dirigé vers l'intérieur de l'insert.

Lorsque cet insert 7a est placé dans l'une des ouvertures 6 pratiquées dans l'âme 2 du collier cervical, le patient peut faire pivoter l'insert 7a entre une première position dans laquelle la paroi diamétrale 10 est orientée dans le sens longitudinal du collier, comme on peut le voir par exemple sur l'insert représenté au centre du collier montré à la figure 1. Dans cette configuration, le maintien que procure le collier est à type souple puisque, lorsqu'une sollicitation s'applique au collier dans une direction transversale à celui-ci, le collier cervical se déforme considérablement. En effet, une sollicitation n'est dissipée que par la portion de mousse située au-dessus et au-dessous de l'ouverture.

En revanche, lorsque l'insert 7a est orienté de telle sorte que sa paroi diamétrale 10 se trouve dans la direction transversale du collier, comme cela est représenté sur les inserts latéraux de la figure 1, une sollicitation dans la direction transversale se traduit par une réponse du collier présentant une plus grande raideur que lorsque l'insert était dans la position précédemment décrite.

En effet, dans cette configuration, le collier ne peut que se déformer faiblement.

La figure 3 représente une autre forme de réalisation de l'insert selon laquelle un insert 7b est constitué d'une paroi périphérique cylindrique 21 et de deux parois intérieures perpendiculaires, le bouton 23 de manipulation étant placé à l'intersection des deux parois intérieures 22a, 22b. Cet insert 7b offre deux possibilités d'élasticité puisqu'il peut être placé dans une position dans laquelle les parois internes 22a, 22b de l'insert 7 sont orientées à 45° de la direction transversale du collier, dans cette position, la sollicitation que le patient peut faire subir au collier est absorbée partiellement, d'une part, par la mousse et, d'autre part, par la paroi extérieure cylindrique qui fléchit.

En revanche, lorsque le patient à l'aide du bouton 23 fait pivoter l'insert 7b de telle sorte que l'une des parois cylindriques se trouve dans la direction transversale du collier cervical, la sollicitation n'est absorbée que par la mousse puisque, dans cette configuration, l'insert 7b ne présente aucune élasticité.

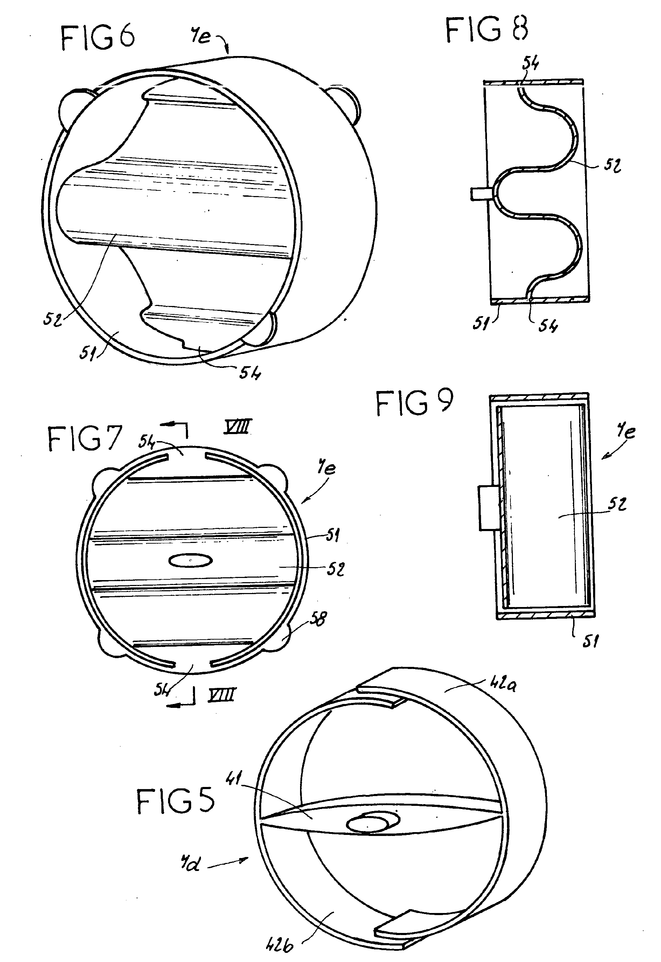

La figure 4 représente une autre forme de réalisation d'un insert 7c selon laquelle l'insert 7c confère trois possibilités d'élasticité au collier. Dans cette forme de réalisation, l'insert 7c est constitué de deux parois diagonales 31 a, 31 b et de deux parois périphériques 32a, 32b en arc de cercle opposées l'une à l'autre par rapport à la ligne d'intersection des parois diagonales 31 a, 31 b.

On note également qu'un bouton 33 de manoeuvre est placé dans le prolongement de la ligne d'intersection des parois diagonales.

Cet insert 7c peut être placé dans une position dans laquelle l'une de ses parois diagonales 31 a, 31b est orientée dans la direction transversale du collier cervical. Dans cette position, le collier présente une dureté maximale puisque les sollicitations ne sont absorbées que par les portions de mousse situées de part et d'autre de l'ouverture. Dans cette position, l'insert 7c ne présente aucune élasticité.

Le patient peut faire pivoter l'insert 7c grâce à son bouton 33 de manoeuvre pour l'amener dans une position dans laquelle les deux parois périphériques 32a, 32b en arc de cercle se trouvent dans la direction transversale du collier cervical. Lorsque l'insert 7c est dans cette position, une sollicitation est absorbée en partie par l'âme 2 et en partie par l'insert 7c dont les parois périphériques en arc de cercle 32a, 32b peuvent se fléchir pour absorber une partie de cette sollicitation.

Dans une troisième configuration, les deux parois périphériques en arc de cercle 32a, 32b se trouvent dans la direction longitudinale du collier cervical. Lorsque l'insert 7c est dans cette orientation, la sollicitation est absorbée exclusivement par l'âme 2 puisque, dans la direction transversale, l'insert 7c n'offre aucune résistance à une sollicitation.

La figure 5 représente une autre forme de réalisation d'un insert 7d offrant deux possibilités d'élasticité au collier cervical. Dans ce mode de réalisation, l'insert 7d est constitué d'une paroi diamétrale 41 portant, à chacune de ses extrémités, deux parois périphériques en arc de cercle 42a, 42b s'étendant sur au moins 180 °, de telle sorte qu'il se produit un léger recouvrement de l'extrémité de chacune des parois périphériques en arc de cercle 42a, 42b de l'insert 7d. Cette dernière disposition permet une rotation plus facile de l'insert dans l'ouverture 6 pratiquée dans l'âme 2 du collier cervical.

L'insert 7d ainsi défini, peut pivoter entre une première position et une seconde position. Dans la première position, la paroi diamétrale 41 est orientée dans la direction transversale du collier. Dans cette configuration, l'insert 7d n'offre qu'une élasticité extrêmement réduite. Dans la seconde position, la paroi diamétrale 41 est orientée dans le sens longitudinal de l'insert. Dans cette dernière position, une sollicitation est absorbée en partie par la mousse et en partie par les parois externes en arc de cercle qui se déforment sous l'effet de cette sollicitation et en absorbent une partie de l'énergie.

Les figures 6 à 9 illustrent une autre forme de réalisation d'un insert 7e offrant également deux possibilités d'élasticité au collier cervical. Dans cette forme de réalisation, l'insert est constitué d'une paroi périphérique cylindrique 51 et d'un disque 52 relié à la paroi cylindrique par deux ponts 54 de matière diamétralement opposés.

Comme cela apparaît à la figure 8 en coupe, le disque 52 présente trois ondulations parallèles. On note également que ces ondulations sont perpendiculaires au plan passant par les deux ponts de matière 54. Lorsque l'insert est orienté par rapport au collier dans une position telle que les ondulations sont dans la direction transversale du collier, une sollicitation appliquée au collier dans cette région n'est absorbée que par la mousse. En effet, dans cette orientation, l'insert ne présente qu'une élasticité extrêmement réduite.

En revanche, lorsque l'insert est pivoté pour se placer dans une position dans laquelle les ondulations sont orientées dans le sens longitudinal, une sollicitation appliquée au collier est absorbée d'une part dans la mousse, et d'autre part, dans l'insert puisque les ondulations permettent à celui-ci de se déformer pour absorber une partie de l'énergie de ladite sollicitation.

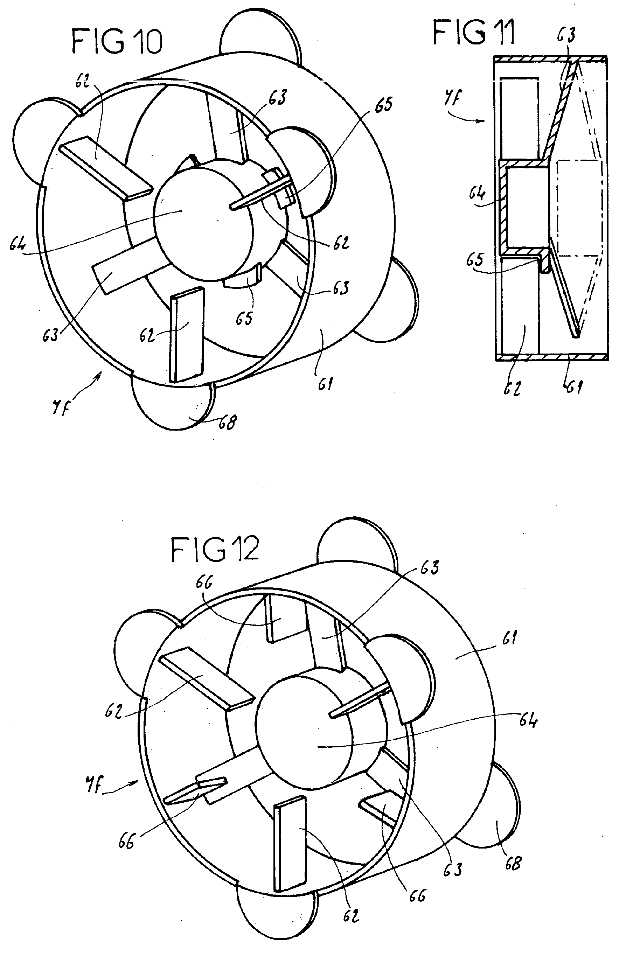

Les figures 10 et 11 représentent une autre forme de réalisation d'un insert 7f. Dans cette forme de réalisation, l'insert 7f est constitué d'une paroi périphérique cylindrique 61 de laquelle s'étendent radialement trois nervures 62 rigides disposées à 120° et trois lames élastiques 63 auxquelles est relié un noyau 64 mobile. Ce noyau 64 est mobile par rapport à l'axe de l'insert entre une position représentée en traits pleins sur la figure 11 dans laquelle le noyau 64 est en appui contre les nervures 62 rigides et une position représentée en traits mixtes sur la figure 11 toujours dans laquelle le noyau 64 est dégagé des nervures 62 rigides et n'est plus retenu à l'insert 7f que par les lames souples 63. On remarque que le noyau 64 vient en appui contre trois butées 65, lorsqu'il est positionné contre les nervures 62.

La figure 12 représente une variante dans laquelle les lames élastiques viennent en appui contre les nervures 66.

On comprend donc que, lorsque le patient déplace le noyau à travers la gaine de matière textile entre c deux positions, il peut placer l'insert dans une position dans laquelle l'insert présentera une rigidité totale et une position dans laquelle il présentera une certaine élasticité.

L'invention fournit ainsi un collier cervical dont le maintien peut être modifié par le patient selon l'évolution de sa pathologie ou suivant les postures qu'il adopte. Bien entendu, l'invention a été décrite à titre d'exemple non limitatif et embrasse toutes les variantes de réalisation

Pour les formes de réalisation représentées aux figures 2, 3, 4 et 5, il peut être envisager de combler les espaces délimités par les parois de l'insert par des éléments de mousse. En effet, il est souhaitable que l'âme ne présente pas des solutions de continuité par lesquelles l'effet thermique pourrait s'échapper.

Les formes de réalisation de l'insert représentées aux figures 6 à 10 présentent des oreilles 58, 68 permettant le maintien de l'insert dans l'âme 2, ces oreilles peuvent être également placées sur les parois extérieures des inserts représentées aux figures 2 à 5.

## Revendications

1. Collier (1) cervical comprenant une âme (2) en matériau élastique et des moyens d'accrochage aptes à maintenir le collier autour du cou d'un patient, **caractérisé en ce que** l'âme présente au moins un logement dans lequel sont intégrés des moyens de sélection d'un module d'élasticité du collier parmi au moins deux modules distincts.

2. Collier cervical selon la revendication 1, **caractérisé en ce que** le logement est une ouverture (6) circulaire pratiquée dans l'épaisseur du collier dans laquelle s'engage l'insert (7a, 7b, 7c, 7d, 7e, 7f).

3. Collier cervical selon la revendication 2, **caractérisé en ce que** l'insert est susceptible d'entrer en rotation dans l'ouverture pour passer d'une orientation de l'insert (7a, 7b, 7c, 7d, 7e, 7f) conférant au collier un premier module d'élasticité à au moins une deuxième orientation de l'insert conférant au collier un deuxième module d'élasticité.

4. Collier cervical selon la revendication 3, **caractérisé en ce que** l'insert est muni d'un bouton (13, 23, 33, 43) dépassant d'une surface latérale de l'âme.

5. Collier cervical selon l'une des revendications 1 à 4, **caractérisé en ce qu'**un insert (7a) est constitué d'une paroi diamétrale (10) portant, à chaque extrémité, deux parois périphériques en arc de cercle (11).

6. Collier cervical selon la revendication 4, **caractérisé en ce que** les parois périphériques en arc de cercle (11) sont munies à chacune de leurs extrémités d'un retour (12) dirigé vers l'intérieur de l'insert (7a).

7. Collier cervical selon l'une des revendications 1 à 4, **caractérisé en ce qu'**un insert (7b) est constitué d'une paroi périphérique cylindrique (21) et de deux parois perpendiculaires (22a, 22b).

8. Collier cervical selon l'une des revendications 1 à 4, **caractérisé en ce qu'**un insert (7c) est constitué de deux parois diagonales (31 a, 31 b) et deux parois périphériques en arc de cercle (32a, 32b) opposées l'une à l'autre par rapport à la ligne d'intersection des parois diagonales (31 a, 31 b).

9. Collier cervical selon l'une des revendications 1 à 4, **caractérisé en ce qu'**un insert (7d) est constitué d'une paroi diamétrale (41) portant à chacune de ses extrémités deux parois périphériques (42a, 42b) en arc de cercle s'étendant chacun sur au moins 180°

10. Collier cervical selon l'une des revendications 5 à 9, **caractérisé en ce que** des éléments de mousse sont engagés dans les espaces délimités pour les parois de chaque insert.

11. Collier cervical selon l'une des revendications 1 à 4, **caractérisé en ce qu'**un insert (7e) est constitué d'une paroi périphérique cylindrique (51) et d'un disque (52) relié à la paroi cylindrique (51) par deux ponts de matière (54) diamétralement opposés, le disque (52) présentant au moins deux coefficients d'élasticité.

12. Collier cervical selon la revendication 11, **caractérisé en ce que** le disque (52) présente au moins une ondulation passant par le centre du disque et perpendiculaire au plan passant par les deux ponts de matière.

13. Collier cervical selon la revendication 12, **caractérisé en ce que** le disque présente trois ondulations parallèles.

14. Collier cervical selon l'une des revendications 1 à 4, **caractérisé en ce qu'**un insert (7f) est constitué d'une paroi périphérique cylindrique (61) périphérique de laquelle s'étendent radialement trois nervures (62) rigides disposées à 120° et trois lames (63) élastiques auxquelles est relié un noyau (64) mobile le long de l'axe de l'insert (7f) entre une position dans laquelle le noyau (64) est en appui contre les nervures (62) rigides et une position dans laquelle le noyau (64) est dégagé de ces dernières et est uniquement retenu par les lames souples.

15. Collier cervical selon l'une des revendications 5 à 14, **caractérisé en ce que** la paroi périphérique (51, 61) de l'insert est muni d'oreilles (58, 68) permettant de retenir l'insert par rapport à l'âme (2).

16. Collier cervical selon l'une des revendications 1 à 15, **caractérisé en ce qu'**une gaine (3) de matière textile recouvre l'âme (2).
